# FASCICULE DE BREVET EUROPEEN

(11) **EP 1 348 704 B1**
(45) Date de publication et mention de la délivrance du brevet: **17.05.2006**
(21) Numéro de dépôt: 03290759.4
(22) Date de dépôt: 26.03.2003
(51) Int. Cl.: C07D 401/12, A61K 31/4439, A61P 25/00, A61P 15/10

(54) **Dérivés d'indole possédant des propriétés antagonistes 5-HT2C, leur procédé de préparation et les compositions pharmaceutiques qui les contiennent**
Indolderivate als 5-HT2C-Antagonisten, Verfahren zu deren Herstellung, sowie diese enthaltende pharmazeutische Zusammensetzungen
Indole derivatives as 5-HT2C antagonists, process for their preparation and pharmaceutical compositions containing them

(30) Priorité: 27.03.2002 FR 0203788
(43) Date de publication de la demande: 01.10.2003
(73) Titulaire: Les Laboratoires Servier, 92415 Courbevoie Cedex (FR)
(72) Inventeur: Lavielle, Gilbert, 78170 La Celle Saint Cloud (FR); Muller, Olivier, 95300 Pontoise (FR); Millan, Mark, 78230 Le Pecq (FR); Gobert, Alain, 92500 Rueil-Malmaison (FR)

(56) Documents cités:
- EP-A- 1 146 044
- WO-A-94/04533
- WO-A-95/01976
- WO-A-95/29177

## Description

La présente invention a pour objet de nouveaux dérivés d'indoline possédant des propriétés antagonistes 5-HT_{2C}, leur procédé de préparation et les compositions pharmaceutiques qui les contiennent.

Les récepteurs 5-HT_{2c} exercent un contrôle inhibiteur sur la transmission dopaminergique et noradrénergique (Neuropharmacology, 1997, 36, 609, J. Psychopharmacol. 2000, 14 (2), 114-138). Ainsi, les antagonistes 5-HT_{2C} sont réputés utiles dans le traitement de nombreuses pathologies du système nerveux central (SNC). Nous pouvons citer sans que cette liste soit complètement exhaustive des désordres tels que l'anxiété (Br. J. Pharmacol., 1996, 117, 427), la dépression (Pharmacol. Biochem. Behav., 1988, 29, 819-820), des désordres impulsifs (Biol. Psych., 1993, 33, 3-14), des dysfonctionnements sexuels (J. Pharmacol., 1997, 11, 72), la maladie de Parkinson (Drug News Perspect., 1999, 12, 477), la migraine (Life Sci., 1994, 54, 641-644), les troubles cognitifs (Neurosci. Biobehav. Rev., 1999, 23, 1111-1125), les troubles du sommeil (Neuropharmacology, 1994, 33, (3/4), 467-471), la schizophrénie (Neurosci. Lett., 1996, 181, 65) et les troubles de l'appétit tels que la boulimie ou l'anorexie (British J. Pharmacol., 1998, 123, 1707-1715).

La présente invention concerne de nouveaux dérivés de l'indoline qui diffèrent des composés des demandes WO 9529177 et WO 9748699, non seulement par l'absence d'un substituant pyridyloxy sur le groupement 3-pyridylaminocarbonyle de l'indoline, mais surtout par la présence d'un groupement benzo fusionné au groupement indoline.

De manière surprenante, ces modifications structurales confèrent aux composés de l'invention des activités pharmacologiques nettement supérieures à celles des composés des demandes WO 9529177 et WO 9748699. Les composés de l'invention se sont révélés être notamment très actif par voie orale.

Ainsi, l'utilisation du radical benzoindoline a permis d'améliorer remarquablement les propriétés pharmacologiques des composés de l'invention.

Plus spécifiquement, la présente invention concerne les composés de formule (I) : 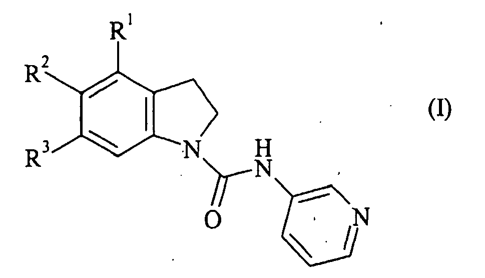 dans laquelle :
R¹, R² forment ensemble un cycle benzo éventuellement substitué par un atome d'halogène ou un groupement alkyle, alkoxy, cyano, nitro, hydroxy, amino, alkylamino, dialkylamino ou trifluorométhyle, et R³ représente un atome d'hydrogène,
ou
R¹ représente un atome d'hydrogène et R², R³ forment ensemble un cycle benzo éventuellement substitué par un atome d'halogène ou un groupement alkyle, alkoxy, cyano, nitro, hydroxy, amino, alkylamino, dialkylamino ou trifluorométhyle,
leurs énantiomères, diastéréoisomères et leurs sels d'addition à un acide ou à une base pharmaceutiquement acceptable,
étant entendu que :
   - le terme alkyle désigne une chaîne hydrocarbonée, linéaire ou ramifiée contenant de 1 à 6 atomes de carbone,
   - le terme alkoxy désigne un groupement alkyle-oxy, linéaire ou ramifié, contenant de 1 à 6 atomes de carbone.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer l'hydroxyde de sodium, l'hydroxyde de potassium, la triéthylamine, la tertbutylamine, etc...

Les composés préférés de l'invention sont ceux pour lesquels R¹, R² forment ensemble un cycle benzo non substitué ou substitué par un groupement choisi parmi méthoxy et cyano, et R³ représente un atome d'hydrogène.

Parmi les composés préférés de l'invention, on peut citer plus particulièrement la *N*-(3-pyridyl)-1,2-dihydro-3*H*-benzo[*e*]indole-3-carboxamide, la 7-méthoxy-*N*-(3-pyridyl)-1,2-dihydro-3*H*-benzo[*e*]indole-3-carboxamide, la 6-cyano-*N*-(3-pyridyl)-1,2-dihydro-3*H-*benzo[e]indole-3-carboxamide et la *N*-(3-pyridyl)-2,3-dihydro-1*H*-benzo[*f*]indole-1-carboxamide.

La présente invention concerne également le procédé de préparation des composés de formule (I) caractérisé en ce que l'on utilise comme produit de départ un dérivé benzoindole de formule (II) : 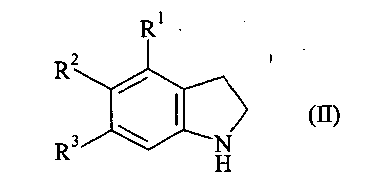 dans laquelle R¹, R² et R³ sont tels que définis dans la formule (I),
qui se condense sous l'action de la chaleur avec un dérivé de formule (III) : 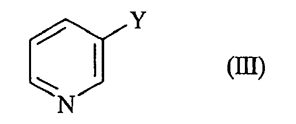 dans laquelle Y représente un groupement -N=C=O ou -C(O)-N₃,
pour conduire au composé de formule (I),
- qui peut être le cas échéant purifié selon une technique classique de purification,
- dont les isomères (diastéréoisomères et énantiomères) sont séparés si nécessaire par une technique classique de séparation,
- que l'on transforme, si on le souhaite, en ses sels d'addition à un acide ou à une base pharmaceutiquement acceptable,
étant entendu que l'indoline de formule (II) est préparée selon des modes opératoires connus, par exemple à partir de dérivé nitronaphtylacétonitrile correspondant.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration oraie, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples qui suivent illustrent l'invention et ne la limitent en aucune façon.

Les structures des composés décrits ont été confirmées par les techniques spectroscopiques et spectrométriques usuelles.

Les produits de départ utilisés sont des produits connus ou préparés selon des modes opératoires connus.

### PREPARATION 1 :

### Stade A : (2-Nitro-1-naphtyl)acétonitrile

Préparer une solution de 53,5 g (0,477 mole) de tertiobutylate de potassium dans 400 ml de diméthylformamide. Refroidir cette solution à -10°C et y additionner en 1 h environ une solution de 40 g de 4-chlorophénoxyacétonitrile (0,24 mole) et 37 g de 2-nitronaphtalène (0,213 mole) dans 200 ml de diméthylformamide. Après 2 heures à -5°C, verser-le tout sur 41 d'eau contenant 1 l d'acide chlorhydrique concentré et extraire la phase aqueuse avec 3 x 500 ml de dichlorométhane. Laver la phase organique avec 300 ml d'eau, la sécher sur sulfate de magnésium, filtrer puis évaporer le solvant.
On obtient 65 g de produit.
Recristalliser ces 65 g. dans un mélange cyclohexane/acétate d'éthyle : 50/50.

### Stade B : 3H-Benzo[e]indole

Hydrogéner à température ambiante sous 4 bars d'hydrogène 33 g de (2-nitro-1-naphtyl)acétonitrile (0,155 mole) dissous dans 630 ml d'éthanol à 10 % d'eau et 6,3 ml d'acide acétique pur : utiliser 19 g de palladium/charbon à 10 %. Après cessation de l'absorption, filtrer le catalyseur, concentrer le solvant sous vide puis reprendre le résidu dans 250 ml de dichlorométhane, laver la phase organique avec 100 ml de solution 0,1 N d'hydroxyde de potassium, puis sécher la phase organique sur sulfate de magnésium, filtrer et concentrer.
Le résidu est purifié par chromatographie sur silice éluant cyclohexane/acétate d'éthyle : 80/20.

### Stade C : 2.3-Dihydro-1H-benzo[e]indole

10 g (0,06 mole) du composé préparé au stade précédent, sont dissous dans 50 ml de tétrahydrofurane. Ajouter à cette solution, à 0°C, 120 ml de complexe borane/THF en solution 1 M dans le tétrahydrofurane, puis 120 ml d'acide trifluoroacétique. Après 30 minutes, ajouter à 0°C, 6 ml d'eau, laisser agiter 15 minutes puis concentrer le tout à sec. Le résidu est repris dans 200 ml de dichlorométhane et lavé avec 200 ml de soude 1 N. La phase organique est séchée sur sulfate de magnésium, filtrée et concentrée.

### PREPARATION 2 : 2,3-Dihydro-1H-benzo[f]indole

Le protocole expérimental pour réduire la 1*H*-benzo[*f*]indole est identique à celui de la Préparation 1, Stade C. La synthèse de la 1*H*-benzo[*f*]indole de départ a été décrite dans la littérature *[Tetrahedron,* 49, 33, 7353 (1993); *Heterocycles,* 24, 7, 1845, (1986)].

### PREPARATION 3 : 2,3-Dihydro-1H-benzo[e]indole-6-carbonitrile

### Stade A : N-(5-Cyano-2-naphtyl)acétamide

A 25 g de *N*-(5,6,7,8-tétrahydronaphtalèn-2-yl)acétamide refroidie à 0°C additionner successivement 70 ml de cyanure de triméthylsilyl pur puis 30 g de dichlorodicyanoquinone dans 70 ml de dichiorométhane. Après 3 heures à température ambiante ajouter à nouveau une solution de 60 g de dichlorodicyanoquinone dans 140 ml de dichlorométhane. Laisser agiter 12 heures à 20°C puis porter 8 heures à 60°C.
Après neutralisation par une solution saturée d'hydrogénocarbonate de sodium, la phase organique est décantée et lavée par de l'eau. Le résidu obtenu après concentration est purifié par chromatographie sur gel de silice en utilisant comme éluant un mélange cyclohexane/acétate d'éthyle : 80/20.

### Stade B : N-(1-Bromo-5-cyano-2-naphtyl)acétamide

A une solution refroidie à -5°C de 50 g (0.238 mole) du produit synthétisé au stade précédent dans 500 ml de dichlorométhane et 25 ml de pyridine, additionner 39,8 g de brome dissous dans 200 ml de dichlorométhane. Laisser ensuite agiter fortement pendant 4 heures à température ambiante, puis diluer avec 500 ml de dichlorométhane, laver la phase organique avec 2 fois 300 ml d'eau, sécher et concentrer. Le résidu est recristallisé dans un mélange dichlorométhane/méthanol : 50/50.

### Stade C : 6-Amino-5-bromo-1-naphtonitrile

Chauffer 6 heures à 80°C un mélange de 12,5 g (0,043 mole) du produit synthétisé au stade précédent, 3,6 g d'hydroxyde de sodium, 195 ml de méthanol et 65 ml d'eau.
Après évaporation du méthanol la phase aqueuse est extraite deux fois par du dichlorométhane. Celui-ci est ensuite séché et évaporé. Le résidu est cristallisé dans un mélange dichlorométhane-méthanol.

### Stade D : 1-Bromo-5-cyano-2-naphtylcarbamate d'éthyle

Additionner à 0°C 19 ml de chloroformiate d'éthyle à une solution de 33 g (0,133 mole) du produit synthétisé au stade précédent dans 200 ml de pyridine. Après 1 heure à 5°C, évaporer le solvant, reprendre le résidu par 500 ml de dichlorométhane, laver la phase organique par 3 fois 100 ml d'acide chlorhydrique 0,1N, puis par 200 ml d'une solution d'hydrogénocarbonate de sodium à 10 % et enfin une fois à l'eau. Le résidu obtenu par évaporation est recristallisé dans un mélange dichiorométnane-métnanoi.

### Stade E : 5-Cvano-1-[(triméthylsilyl)éthynyl]-2-naphtylcarbamate d'éthyle

Dans un réacteur en acier mélanger 14,1 g (0,044 mole) du produit synthétisé au stade précédent, 11 ml de triméthylsilylacétylène, 13ml de triéthylamine, 670 mg d'iodure cuivreux et 1,54 g de dichloro bis(triphénylphosphine)palladium. Fermer ensuite le réacteur et porter le mélange réactionnel à 80°C pendant 4 heures. Diluer avec 200 ml de dichlorométhane et 100 ml d'eau, filtrer le tout, décanter la phase organique, la sécher et évaporer le solvant sous vide. Le résidu obtenu est purifié par chromatographie sur gel de silice en utilisant comme éluant un mélange cyclohexane/acétate d'éthyle : 90/10 puis cristallisation dans le même solvant.

### Stade F : 3H-benzo[e]indole-6-carbonitrile

A une solution de 2,74 g de sodium dans 280 ml d'éthanol sec ajouter 10 g (0,0297 mole) du produit synthétisé au stade précédent et porter le tout une heure à reflux. Après évaporation du solvant, le résidu est repris dans 200 ml de dichlorométhane et la phase organique est lavée par 200 ml d'eau. Après évaporation le résidu est purifié par chromatographie sur gel de silice en utilisant comme éluant un mélange cyclohexane/acétate d'éthyle : 80/20.

### Stade G : 2,3-dihydro-1H-benzo[e]indole-6-carbonitrile

Le protocole expérimental pour réduire le 3*H*-benzo[e]indole-6-carbonitrile est identique à celui de la Préparation 1, Stade C.

### PREPARATION 4 : 7-Métboxv-2,3-dibydro-1H-benzo[e]lindole

### Stade A : 6-Méthoxy-3,4-dihydro-1(2H)-naphtalénone oxime

Solubiliser 100 g (0,57 mol) de 6-méthoxy-1-tétralone dans 2,5 1 d'un mélange éthanol/eau : 80/20. Ensuite sont additionnés à température ambiante 85 g (1,04 mol) d'acétate de sodium et 43 g (0,62 mol) de chlorhydrate d'hydroxylamine. Porter au reflux 4 heures la suspension. Diluer le milieu par 51 d'eau et extraire à l'éther éthylique, laver à l'eau, sécher sur sulfate de magnésium, filtrer. Après évaporation du solvant on obtient 99 g d'un solide beige.

### Stade B : 2-Amino-6-méthoxy-3,4-dihydro-1(2H)-naphtalénone

Solubiliser 50 g (0,26 mol)) du produit synthétisé au stade précédent dans 185 ml de pyridine, puis additionner à température ambiante 54,9 g (0,29 mol) de chlorure de paratoluènesulfonyle. Après 24 heures verser sur de la glace puis filtrer le précipité. Reprendre le précipité dans du dichlorométhane et laver à l'eau, sécher la phase organique sur sulfate de magnésium, filtrer. Après évaporation du solvant on obtient 89 g d'un solide jaune (produit intermédiaire 1).

Ajouter 7,48 g (0,32 mol) de sodium dans un mélange toluène/éthanol : 720/148 ml. Après dissolution, diluer par 940 ml de toluène et additionner rapidement 117 g (0,34 mol) du produit intermédiaire 1. Après 24 heures à température ambiante, filtrer le paratoluènesulfonate de sodium rincer au toluène, puis verser la solution organique sur une solution d'acide chlorhydrique 10 % (1,1 1). Décanter, extraire une fois à l'eau puis évaporer la phase aqueuse. Reprendre le résidu dans de l'éthanol puis filtrer le précipité. On obtient 46,5 g d'un solide beige sous forme chlorhydrate.

### Stade C : N-Ethyl-N'-(6-méthoxy-I-oxo-1,2,3,4-tétrahydro-2-naphtalényl)urée

Verser 4,77 g (0,044 mol) de chloroformiate d'éthyle à 0°C sur 5 g (0,022 mol) de 2-amino-6-méthoxy-3,4-dihydro-2*H*-naphtalèn-1-one en solution dans la pyrydine. Après deux heures à température ambiante concentrer la pyridine, reprendre par du dichlorométhane puis laver la phase organique par une solution d'acide chlorhydrique 0,1N puis une solution saturée d'hydrogénocarbonate de sodium et de l'eau, sécher sur sulfate de magnésium, filtrer, évaporer le solvant. 5,48 g d'un solide orange sont obtenus.

### Stade D : N-Ethyl-N'-(6-méthoxy-1,2,3,4-tétrahydro-2-naphtalènyl)urée

Hydrogéner à 60°C sous pression atmosphérique 98 g (0,37 mole) du produit précédemment préparé au Stade C, dissout dans 1,51 d'éthanol avec 10 g de palladium/charbon à 5 %. Après cessation de l'absorption, filtrer le catalyseur, concentrer le solvant sous vide. On obtient 88,2 g d'une huile.

### Stade E : N-Ethyl-N'-(6-méthoxy-2-naphtyl)urée

Solubiliser 7,42 g (0,0298 mol) du produit synthétisé au stade précédent, dans 100 ml de toluène. Ajouter 13,51 g (0,0595 mol) de dichlorodicyanoquinone et porter au reflux 30 minutes. Filtrer à température ambiante le précipité, rincer au toluène puis évaporer le solvant. Le résidu est purifié par chromatographie sur gel de silice en utilisant comme éluant du dichlorométhane pur. On obtient 4 g d'un solide gris

### Stade F : 6-Méthoxy-2-naphtylamine

Solubiliser 3,5 g du produit synthétisé au stade précédent, dans 65 ml d'éthanol ensuite ajouter une solution d'hydroxyde de potassium dans 65 ml d'eau. Après 4 heures de reflux filtrer à température ambiante le précipiter formé. Reprendre le brut par du dichlorométhane et laver à l'eau jusqu'à neutralité, sécher sur sulfate de magnésium, filtrer le précipité puis évaporer le solvant. On obtient 2,02 g d'un solide orange.

### Stade G : 1-Iodo-6-méthoxy-2-naphtylamine

28,2 g (0,16 mol) du produit synthétisé au stade précédent, sont dissous dans un mélange dichlorométhane/méthanol : 1500/620 ml. Ajouter à cette solution à température ambiante 56,7 g (0,16 mol) de benzyltriméthylammonium dichloroiodate et 21,2 g (0,212 mol) de carbonate de calcium. Après 30 minutes filtrer l'insoluble puis reprendre la phase organique par une solution de bisulfite de sodium à 10 % et extraire à l'éther éthylique. Sécher sur sulfate de magnésium, filtrer puis évaporer. Le résidu est purifié par chromatographie sur gel de silice en utilisant comme éluant un mélange cyclohéxane/acétate d'éthyle : 70/30.

### Stade H: 1-Iodo-6-méthoxy-2-naphtylcarbamate d'éthyle

La transformation du 1-iodo-6-méthoxy-2-naphthylamine s'effectue en appliquant la méthode décrite au Stade C.

### Stade I : 6-Méthoxy-1-[(triméthylsilyl)éthynyl]-2-naphtylcarbamate d'éthyle

La transformation 1-iodo-6-méthoxy-2-naphtylcarbamate d'éthyle s'effectue en appliquant la méthode décrite dans la Préparation 3, Stade E.

### Stade J : 7-Méthoxy-3H-benzo[e]indole

La transformation du composé du stade précédent s'effectue en appliquant la méthode décrite dans la Préparation 3, Stade F.

### Stade K : 7-Méthoxy-2,3-dihydro-1H-benzo[e]indole

Le protocole expérimental pour réduire le 7-méthoxy-3*H*-benzo[*e*]indole est identique à celui de la Préparation 1, Stade C.

### EXEMPLE 1 : N-(3-Pyridinyl)-1,2-dihydro-3H-benzo[e]indole-3-carboxamide

Dissoudre 1,92 g (0,013 mole) d'azoture de nicotinoyl dans 250 ml de toluène et porter le tout à reflux 30 minutes. Refroidir alors à 15°C et additionner rapidement une solution de 2,4 g (0,013 mole) du produit synthétisé dans la Préparation 1. Après quelques minutes, filtrer le précipité formé, le réempâter à l'éther éthylique et recristalliser le résidu dans 130 ml d'éthanol. Filtrer les cristaux.

Point de fusion : 183-186°C.

### EXEMPLE 2 : Chlorhydrate du N-(3-pyridinyl)-2,3-dihydro-1H-benzo[f]indole-1-carboxamide

Le protocole expérimental est identique à celui de l'Exemple 1 en partant du produit de la Préparation 2.

Point de fusion : 235-240°C

### EXEMPLE 3 : Chlorhydrate de 6-cyano-N-(3-pyridinyl)-1,2-dihydro-3H-benzo[e]-indole-3-carboxamide'

Le protocole expérimental est identique à celui de l'Exemple 1 en partant du produit de la Préparation 3.

Point de fusion : 265°C dec.

### EXEMPLE 4 : Chlorhydrate de 7-méthoxy-N-(3-pyridinyl)-1,2-dihydro-3H-benzo[e]-indole-3-carboxamide

Le protocole expérimental est identique à celui de l'Exemple 1 en partant du produit de la Préparation 4.

Point de fusion : >270°C dec.

### ETUDE PHARMACOLOGIQUE

### EXEMPLE 5 : Mesure de la concentration extracellulaire de dopamine et de noradrénaline au niveau du cortex frontal chez le rat vigile

### Méthode :

Dialyse. La chirurgie est réalisée sous anesthésié au pentobarbital (60 mg/kg, i.p.). Les rats sont placés dans un appareil stéréotaxique de Kopf et un guide de canule (CMAMicrodialyse AB, Stockholm, Suède) implanté dans le cortex frontal aux coordonnées: antéropostérieur: + 2.2, latéral: ± 0.6, déviation ventrale: - 0.2. Les rats, mis en cage individuelle, récupèrent de l'anesthésie pendant 5 jours. Le jour de la dialyse, une sonde en cuprophan CMA/11 (4 mm de long, 0,24 mm de diam. ext.) est introduite dans le guide et perfusée à 1 µl/min. par une solution de NaCl: 147,2 mM; KCl: 4 mM; CaCl₂: 2,3 mM, amenée grâce à un tampon phosphate à pH 7,3. Deux heures après l'implantation, des échantillons de dialysat sont collectés toutes les 20 min. pendant 4 heures. Trois échantillons basaux sont collectés avant l'administration de la drogue.

*Chromatographie.* La dopamine (DA) et la noradrenaline (NA) sont simultanément mesurées de la manière suivantes: 20 µl d'échantillon de dialysat sont dilués avec 20 µl de phase mobile (NaH₂PO₄: 75 mM, EDTA: 20 µM, sodium décanesulphonate: 1 mM, méthanol : 17,5 %, triéthylamine 0,01 %, pH : 5,70) et 33 µl sont analysés par HPLC en utilisant pour séparation, une colonne phase inverse (hypersil C18, 150 x 4.6 mm, taille des particules, 5 µm) thermostatée à 43°C et pour quantification, un détecteur coulométrique (ESA5014, Coulochem II, ESA, Chelmstord, USA). Le potentiel de la première électrode étant de - 90 mV (réduction) et de la seconde de + 280 mV (oxydation). Le débit de la phase mobile est de 2 ml/min.. Les limites de sensibilité pour la DA et la NA sont respectivement de 0,1 et 0,2 pg.

### Résultats :

Le composé de l'exemple 1 (40,0 mg/kg, P.O.) induit une augmentation importante de la concentration extracellulaire de DA et de NA dans les dialysats recueillis dans le cortex frontal des rats vigiles. Les données sont exprimées comme la moyenne ± E.S.M.. Les niveaux de DA et NA sont exprimées par rapport à la moyenne des valeurs avant l'administration de la drogue. Les effets de la drogue sont comparés (t test non apparié) à ceux obtenus chez les animaux traités par le solvant au temps 30 min. post administration. **DA: solvant = + 3,9 ± 9,8 *versus* drogue = + 95,0 ± 12,9 et NA: solvant = + 8,7 ± 11,9 *versus* drogue = + 118,2 ± 16,7.**

### EXEMPLE 6 : Test des érections péniennes provoquées par le Ro 60-0175 (1,25 mg/kg, s.c.) chez le rat

### Méthode :

Le test permet d'évaluer la capacité d'agents pharmacologiques à inhiber les érections péniennes provoquées par l'administration de Ro 60-0175, un agoniste sélectif 5-HT_{2C}. Une inhibition est donc prédictive d'une activité antagoniste au niveau des récepteurs 5-HT_{2C}. Des rats mâles de souche Wistar (Iffa-Credo, L'Arbresle, France), pesant 120-140 g le jour de l'expérience, sont placés individuellement dans des boites d'observation (7,5 x 18 x 30 cm) en plexiglass, juste après avoir reçu le produit ou son véhicule. Trente minutes plus tard, les animaux reçoivent le Ro 60-0175 (1,25 mg/kg, s.c.) et le nombre d'érections effectuées lors des 30 minutes suivantes est comptabilisé.

### Résultats :

| **Dose du composé de l'Exemple 1** mg/kg p.o. | **% inhibition** | **DI**_{**50**} mg/kg p.o. |
|---|---|---|
| 0,25 | 12 | |
| 0,63 | 16 | |
| 1,00 | 50 | 1,16 |
| 2,5 | 88 | |
| 10,0 | 100 | |

| | | |
|---|---|---|
| **DI**_{**50**} **= dose inhibitrice**_{**50**}**.** | | |

### EXEMPLE 7 : Test d'interaction sociale chez le rat

### Méthode :

Le test permet d'évaluer l'activité anxiolytique potentielle des produits. Des rats mâles de souche Sprague-Dawley (Charles River, Saint-Aubin-les-Elbeuf, France), pesant 240-260 g à leur arrivée, sont isolés en cages individuelles pendant 5 jours dans une animalerie faiblement éclairée (3-10 lux). Le test prend place le dernier jour et consiste à placer les animaux par paire appariée selon le poids (± 5 g) dans une enceinte fortement éclairée (300 lux) pendant 10 minutes. Le temps passé par les animaux à interagir entre eux (toilettage, poursuite, ...) est comptabilisé. Les animaux reçoivent le produit ou son véhicule 1 heure avant le test. chaque couple reçoit le même traitement. L'effet anxiolytique d'un produit se traduit par une durée d'interaction élevée comparativement à celle d'animaux recevant le véhicule.

### Résultats :

| **Dose du composé de l'Exemple 1** mg/kg p.o. | **durée des interactions sociales (s)** moyenne ± e.s.m. (n) |
|---|---|
| *0* | *183 ± 7(12)* |
| 0,16 | 187 ± 11 (5) |
| 0,63 | 228 ± 11 (5)* |
| 2,5 | 242 ± 15 (5) * |
| 10,0 | 233 ± 10 (5)* |

| | |
|---|---|
| **e.s.m. : erreur standard à la moyenne** | |
| n : nombre de rats | |
| Comparaison *versus* véhicule (test de Dunnett) : * p< 0,05. | |

### EXEMPLE 8 : Test de Conflit de Vogel

### Méthode :

Le test se déroule dans une cage plastique transparente (32 x 25 x 30 cm) située dans une enceinte insonorisée et ventilée. La cage comporte une grille en acier chromé. L'embout métallique du flacon contenant la boisson pénètre dans la cage à une hauteur de 6 cm au-dessus du plancher métallique. Le plancher et l'embout du flacon sont reliés par des câbles électriques à un appareil "Anxiomètre" (Columbus Instruments, Ohio, USA) qui assure l'enregistrement des lèchements de l'animal et contrôle l'administration des chocs électriques.

Pendant les 4 jours précédant ie test , les rats (Wistar mâles, poids 250-270 g), (3/cage) n'ont qu'une seule heure par jour d'accès à la boisson. La veille du test, les animaux sont isolés dans des cages sur grille, sans boisson ni nourriture, juste après leur dernière heure d'accès à la boisson. Le test a lieu le 5^{e} jour. Le jour du test, l'animal reçoit par gavage (p.o.), le solvant (suspension d' eau distillée + Tween 80) (Témoin) ou le produit, 30 minutes avant d'être placé dans la cage Test. La session débute dès que l'animal a effectué 20 lèchements et a reçu un premier choc électrique (entre embout et plancher métallique), (courant constant, durée 0.5 sec, intensité 0.3 mA). Ensuite, l'animal reçoit un choc électrique chaque fois qu'il effectue 20 lèchements, pendant une période de 3 minutes. Les animaux n'ayant pas effectué les 20 premiers lèchements de la session au bout de 5 min, sont retirés du test. Les résultats sont les nombres de lèchements effectués par l'animal pendant les 3 minutes du Test.

### Résultats :

| **Doses du composé de l'Exemple 1** mg/kg, p.o. | **Lèchements punis (1 choc / 20 lèchements)** Moyenne ± e s.m. (n) |
|---|---|
| *0* | *77.1 ±14.7(9)* |
| 2.5 | 92.2 ± 20.7 (5) |
| 10.0 | 145.2 ± 46.3 (5) |
| 40.0 | 324.4 ± 70.7 (10)* |

| | |
|---|---|
| **e.s.m. : erreur standard à la moyenne** | |
| n : nombre de rats | |
| Comparaison *versus* véhicule (test de Dunnett) : * p< 0,05. | |

### EXEMPLE 9 : Composition pharmaceutique

### Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 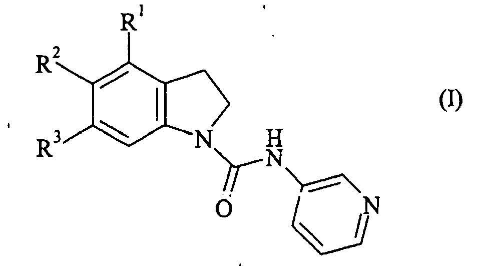 dans laquelle :
R¹, R² forment ensemble un cycle benzo éventuellement substitué par un atome d'halogène ou un groupement alkyle, alkoxy, cyano, nitro, hydroxy, amino, alkylamino, dialkylamino ou trifluorométhyle, et R³ représente un atome d'hydrogène,
ou
R¹ représente un atome d'hydrogène et R², R³ forment ensemble un cycle benzo éventuellement substitué par un atome d'halogène ou un groupement alkyle, alkoxy, cyano, nitro, hydroxy, amino, alkylamino, dialkylamino ou trifluorométhyle,
leurs énantiomères, diastéréoisomères sels d'addition à un acide ou à une base pharmaceutiquement acceptable,
étant entendu que :
- le terme alkyle désigne une chaîne hydrocarbonée, linéaire ou ramifiée contenant de 1 à 6 atomes de carbone,
- le terme alkoxy désigne un groupement alkyle-oxy, linéaire ou ramifié, contenant de 1 à 6 atomes de carbone.

2. Composés de formule (I), selon la revendication 1, pour lesquels R¹ et R² forment ensemble un cycle benzo non substitué ou substitué par un groupement choisi parmi méthoxy et cyano et R³ représente un atome d'hydrogène, et leurs sels d'addition à un acide ou à une base pharmaceutiquement acceptable.

3. Composés de formule (I), selon une des revendications 1 ou 2 qui est la *N*-(3-pyridyl)-1,2-dihydro-3*H*-benzo[*e*]indole-3-carboxamide et ses sels d'addition à un acide ou à une base pharmaceutiquement acceptable.

4. Composé de formule (I), selon une des revendications 1 ou 2 qui est la 7-méthoxy-*N*-(3-pyridyl)-1,2-dihydro-3*H*-benzo[*e*]indole-3-carboxamide et ses sels d'addition à un acide ou à une base pharmaceutiquement acceptable.

5. Composé de formule (I), selon une des revendications 1 ou 2 qui est la 6-cyano-*N*-(3-pyridyl)-1,2-dihydro-3*H*-benzo[*e*]indole-3-carboxamide et ses sels d'addition à un acide ou à une base pharmaceutiquement acceptable.

6. Composé de formule (I), selon la revendication 1 qui est la *N*-(3-pyridyl)-2,3-dihydro-1*H*-benzo[*f*]indole-1-carboxamide et ses sels d'addition à un acide ou à une base pharmaceutiquement acceptable.

7. Procédé de préparation d'un composé de formule (I), selon la revendication 1, **caractérisé en ce que** nous utilisions comme produit de départ un dérivé benzoindole de formule (II) : 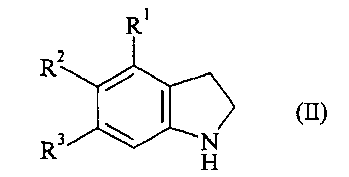 dans laquelle R¹, R² et R³ sont tels que définis dans la formule (I),
qui se condense sous l'action de la chaleur avec un dérivé de formule (III) : 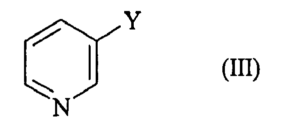 dans laquelle Y représente un groupement -N=C=O ou -C(O)-N₃,
pour conduire au composé de formule (I),
• qui peut être le cas échéant purifié selon une technique classique de purification,
• dont les isomères (diastéréoisomères et énantiomères) sont séparés si nécessaire par une technique classique de séparation,
• que l'on transforme, si on le souhaite, en ses sels d'addition à un acide ou à une base pharmaceutiquement acceptable,
étant entendu que l'indoline de formule (II) est préparée selon des modes opératoires connus, par exemple à partir de dérivé nitronaphtylacétonitrile correspondant.

8. Compositions pharmaceutiques contenant comme principe actif au moins un composé selon l'une quelconque des revendications 1 à 6, seul ou en combinaison avec un ou plusieurs excipients ou véhicules inertes non toxiques, pharmaceutiquement acceptables.

9. Compositions pharmaceutiques selon la revendication 8 contenant au moins un principe actif selon l'une quelconque des revendications 1 à 6 utiles pour la fabrication de médicaments antagonistes 5-HT_{2C}.

10. Compositions pharmaceutiques selon la revendication 8 contenant au moins un principe actif selon l'une quelconque des revendications 1 à 6 utiles pour la fabrication de médicaments utiles dans le traitement de la dépression, de l'anxiété, des désordres impulsifs, de la schizophrénie, de la maladie de Parkinson, la migraine, des troubles cognitifs, des troubles de la libido, et des dysfonctionnements sexuels, des troubles du sommeil, et des troubles de l'appétit tels que la boulimie ou l'anorexie.

## Claims

1. Compounds of formula (I) : 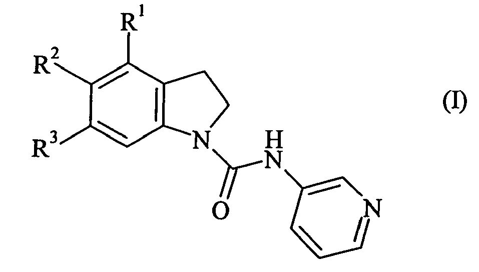 wherein:
R¹ and R² together form a benzo ring optionally substituted by a halogen atom or an alkyl, alkoxy, cyano, nitro, hydroxy, amino, alkylamino, dialkylamino or trifluoromethyl group, and R³ represents a hydrogen atom,
or
R¹ represents a hydrogen atom, and R² and R³ together form a benzo ring optionally substituted by a halogen atom or an alkyl, alkoxy, cyano, nitro, hydroxy, amino, alkylamino, dialkylamino or trifluoromethyl group,
their enantiomers and diastereoisomers, and addition salts thereof with a pharmaceutically acceptable acid or base,
it being understood that :
- the term "alkyl" denotes a linear or branched hydrocarbon chain containing from 1 to 6 carbon atoms,
- the term "alkoxy" denotes a linear or branched alkyl-oxy group containing from 1 to 6 carbon atoms.

2. Compounds of formula (I), according to claim 1, wherein R¹ and R² together form a benzo ring which is unsubstituted or substituted by a group selected from methoxy and cyano, and R³ represents a hydrogen atom, and addition salts thereof with a pharmaceutically acceptable acid or base.

3. Compound of formula (I), according to either claim 1 or claim 2, which is *N*-(3-pyridyl)-1,2-dihydro-3H-benzo[e]indole-3-carboxamide and addition salts thereof with a pharmaceutically acceptable acid or base.

4. Compound of formula (I), according to either claim 1 or claim 2, which is 7-methoxy-*N-*(3-pyridyl)-1,2-dihydro-3*H*-benzo[e]indole-3-carboxamide and addition salts thereof with a pharmaceutically acceptable acid or base.

5. Compound of formula (I), according to either claim 1 or claim 2, which is 6-cyano-N-(3-pyridyl)-1,2-dihydro-3*H*-benzo[e]indole-3-carboxamide and addition salts thereof with a pharmaceutically acceptable acid or base.

6. Compound of formula (I), according to claim 1, which is *N*-(3-pyridyl)-2,3-dihydro-1*H-*benzo[*f*]indole-1-carboxamide and addition salts thereof with a pharmaceutically acceptable acid or base.

7. Process for the preparation of a compound of formula (I), according to claim 1, **characterised in that** there is used as starting material a benzoindole compound of **formula (II) :** 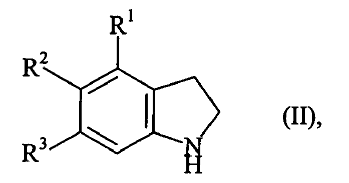 wherein R¹, R² and R³ are as defined for formula (I),
which is condensed, under the action of heat, with a compound of formula (III) : 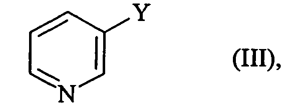 wherein Y represents a group -N=C=O or -C(O)-N₃,
to yield the compound of formula (I),
• which may be purified, if necessary, according to a conventional purification technique,
• which is separated, if necessary, into its isomers (diastereoisomers and enantiomers) by a conventional separation technique,
• which is converted, if desired, into addition salts thereof with a pharmaceutically acceptable acid or base,
it being understood that the indoline of formula (II) is prepared according to known procedures, for example starting from the corresponding nitronaphthylacetonitrile compound.

8. Pharmaceutical compositions comprising as active ingredient at least one compound according to any one of claims 1 to 6, alone or in combination with one or more inert, nontoxic, pharmaceutically acceptable excipients or carriers.

9. Pharmaceutical compositions according to claim 8, comprising at least one active ingredient according to any one of claims 1 to 6, for use in the manufacture of 5-HT_{2C} antagonist medicaments.

10. Pharmaceutical compositions according to claim 8, comprising at least one active ingredient according to any one of claims 1 to 6, for use in the manufacture of medicaments for use in the treatment of depression, anxiety, impulsive disorders, schizophrenia, Parkinson's disease, migraine, cognitive disorders, disorders of the libido and sexual dysfunctions, sleep disorders, and appetite disorders such as bulimia and anorexia.

## Patentansprüche

1. Verbindungen der Formel (I): 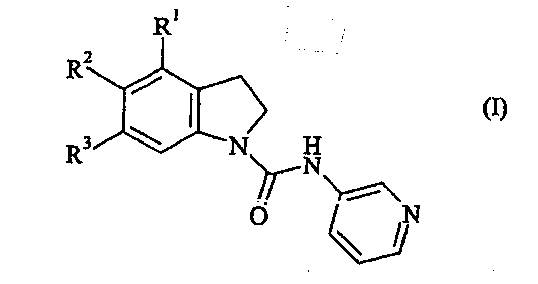 in der:
R¹ und R² gemeinsam einen Benzocyclus bilden, der gegebenenfalls durch ein Halogenatom oder eine Alkyl-, Alkoxy-, Cyano-, Nitro-, Hydroxy-, Amino-, Alkylamino-, Dialkylamino- oder Trifluormethyl-gruppe substituiert ist und R³ ein Wasserstoffatom bedeutet,
oder
R¹ ein Wassertsoffatom bedeutet und R² und R³ gemeinsam einen Benzocyclus bilden, der gegebenenfalls durch ein Halogenatom oder eine Alkyl-, Alkoxy-, Cyano-Nitro-, Hydroxy-, Amino-, Alkylamino-, Dialkylamino- oder Trifluormethylgruppe substituiert ist,
deren Enantiomere, Diastereoisomere und Additionssalze mit einer pharmazeutisch annehmbaren Säure oder Base,
mit der Maßgabe, daß:
- der Begriff Alkyl für eine geradkettige oder verzweigte Kohlenwasserstoffkette steht, die 1 bis 6 Kohlenstoffatome enthält,
- der Begriff Alkoxy für eine geradkettige oder verzweigte Alkyloxygruppe steht, die 1 bis 6 Kohlenstoffatome enthält.

2. Verbindungen der Formel (I) nach Anspruch 1, worin R¹ und R² gemeinsam einen Benzocyclus bilden, der nicht substituiert ist oder durch eine Gruppe ausgewählt aus Methoxy und Cyano substituiert ist, und R³ ein Wasserstoffatom bedeutet, sowie deren deren Additionssalze mit einer pharmazeutisch annehmbaren Säure oder Base.

3. Verbindungen der Formel (I) nach einem der Ansprüche 1 oder 2, nämlich *N*-(3-Pyridyl)-1,2-dihydro-3*H*-benzo[e]indol-3-carboxamid und dessen Additionssalze mit einer pharmazeutisch annehmbaren Säure oder Base.

4. Verbindung der Formel (I) nach einem der Ansprüche 1 oder 2, nämlich 7-Methoxy-N-(3-Pyridyl)-1,2-dihydro-3H-benzo[e]indol-3-carboxamid und dessen Additionssalze mit einer pharmazeutisch annehmbaren Säure oder Base.

5. Verbindung der Formel (I) nach einem der Ansprüche 1 oder 2, nämlich 6-Cyano-*N*-(3-Pyridyl)-1,2-dihydro-3H-benzo[e]indol-3-carboxamid und dessen Additionssalze mit einer pharmazeutisch annehmbaren Säure oder Base.

6. Verbindung der Formel (I) nach Anspruch 1, nämlich N-(3-Pyridyl)-2,3-dihydro-1*H*-benzo[*f*]indol-1-carboxamid und dessen Additionssalze mit einer pharmazeutisch annehmbaren Säure oder Base.

7. Verfahren zur Herstellung einer Verbindung der Formel (I) nach Anspruch 1, **dadurch gekennzeichnet, daß** man als Ausgangsprodukt ein Benzoindolderivat der Formel (II) verwendet: 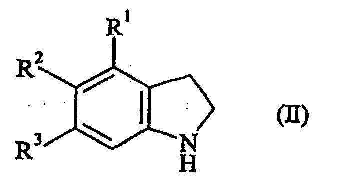 in der R¹, R² und R³ die bezüglich der Formel (I) angegebenen Bedeutungen besitzen,
welche man unter Wärmeeinwirkung mit einem Derivat der Formel (III) kondensiert: 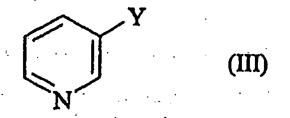 in der Y eine Gruppe -N=C=O oder -C(O)-N₃ darstellt,
zur Bildung der Verbindung der Formel (I),
. welche gegebenenfalls mit Hilfe einer klassischen Reinigungsmethode gereinigt werden kann,
. deren Isomere (Diastereoisomere und Enantiomere) erforderlichenfalls mit Hilfe einer klassischen Trennmethode getrennt werden
. und welche man gewünschtenfalls in ihre Additionssalze mit einer pharmazeutisch annehmbaren Säure oder Base überführt,
mit der Maßgabe, daß das Indolin der Formel (II) mit Hilfe bekannter Verfahrensweisen hergestellt wird, beispielsweise ausgehend von dem entsprechenden Nitronaphthylacetonitril-Derivat.

8. Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien oder Hilfsstoffen.

9. Pharmazeutische Zubereitungen nach Anspruch 8, enthaltend mindestens einen Wirkstoff nach einem der Ansprüche 1 bis 6, nützlich für die Herstellung von gegenüber 5-HT_{2c} antagonistischen Arzneimitteln.

10. Pharmazeutische Zubereitungen nach Anspruch 8, enthaltend mindestens einen Wirkstoff nach einem der Ansprüche 1 bis 6 für die Herstellung von Arzneimitteln, die nützlich sind bei der Behandlung von Depressionen, der Angst, von impulsiven Störungen, der Schizophrenie, der Parkinsonschen Krankheit, von Migräne, von Erkenntnisstörungen, von Libidostörungen und sexuellen Dysfunktionen, von Schlafstörungen und Appetitstörungen, wie Bulimie oder Anorexie.
